# FASCICULE DE BREVET EUROPEEN

(11) **EP 4 355 194 B1**
(45) Date de publication et mention de la délivrance du brevet: **09.04.2025**
(21) Numéro de dépôt: 22733640.1
(22) Date de dépôt: 17.06.2022
(51) Int. Cl.: A61B 3/04, A61B 3/113, A61B 3/00

(54) **PROCÉDÉ D'ESSAYAGE DE LUNETTES VIRTUELLES**
VERFAHREN ZUR ANPASSUNG EINER VIRTUELLEN BRILLE
METHOD FOR FITTING VIRTUAL GLASSES

(30) Priorité: 18.06.2021 FR 2106482
(43) Date de publication de la demande: 24.04.2024
(73) Titulaire: ACEP France, 75017 Paris (FR)
(72) Inventeur: GRILLON, Benoit, 45000 ORLEANS (FR); SAYAG, Jean-philippe, 75017 PARIS (FR)
(74) Mandataire: Oudin, Stéphane
(86) Numéro de dépôt international: PCT/EP2022/066607
(87) Numéro de publication internationale: WO 2022/263654

(56) Documents cités:
- EP-A1- 3 410 178
- WO-A1-2019/007939
- FR-A1- 3 069 687
- US-A1- 2016 246 078
- US-A1- 2019 033 624
- US-A1- 2020 219 326
- US-A1- 2021 088 811

## Description

### Domaine technique de l'invention

La présente invention concerne un procédé d'essayage d'une paire de lunettes virtuelle et un procédé permettant de mesurer les paramètres pour la préparation d'une lentille ophtalmique en vue de son montage dans une paire de lunettes réelle similaire à la paire de lunettes virtuelles essayée.

### Etat de la technique

Après la prescription de verres correcteurs par l'ophtalmologue, le patient choisit une monture dans laquelle les verres vont être montés.

Habituellement, le choix des montures se fait chez un opticien qui va ensuite mesurer différents paramètres propres à la monture, au visage du patient et à son attitude en vision de près et/ou de loin. Parmi ces paramètres, on peut notamment citer la distance du verre à l'œil, l'angle de galbe de la monture, l'angle d'inclinaison de la monture, les écarts pupillaires et les hauteurs pupillaires. La mesure de ces paramètres permet de préparer des verres ophtalmiques parfaitement adaptés à la monture et aux patients.

Récemment, des dispositifs ont été développés pour calculer automatiquement les paramètres ophtalmiques à partir d'images du visage de l'utilisateur portant la monture. Ces dispositifs prennent la forme de colonnes fixes ou de tablettes portables comprenant au moins une caméra et un ordinateur apte à traiter l'image saisie par la caméra pour calculer les paramètres souhaités. Souvent, la monture essayée est associée temporairement à un repère de taille prédéfini permettant d'améliorer la précision de la mesure.

Depuis quelques années, l'essayage réel de la monture chez l'opticien est concurrencé par des moyens d'essayage de monture virtuelle en boutique ou au domicile. Typiquement, ces moyens d'essayage comprennent une caméra qui saisit en temps réel l'image de l'utilisateur et la transmet, associé à une monture virtuelle, sur un écran d'affichage. Ces moyens sont associés à un programme informatique qui est capable d'extraire de l'image de l'utilisateur, des données telles que la position du visage, la position des yeux de l'utilisateur et de calculer une scène virtuelle comprenant le visage de l'utilisateur sur lequel est surimposée une paire de lunettes 3D choisie par l'utilisateur dans une banque de données. Des procédés standards d'essayage de lunettes virtuelles sont décrits dans les documents US 2019/033624 A1, WO 2019/007939 A1, US 2020/219326 A1 et US 2021/088811 A1. US 2019/033624 A1 décrit, en particulier, un procédé d'essayage de lunettes virtuelles comprenant les étapes consistant à: disposer sur le visage d'un utilisateur une paire de lunettes réelle; cartographier le visage de l'utilisateur par un moyen de cartographie 3D et mesurer les paramètres ophtalmologiques dudit utilisateur à partir du modèle 3D du visage de l'utilisateur construit à l'étape précédante.

Cet essayage virtuel est totalement délocalisable car il est possible de le mettre en œuvre sur le smartphone ou l'ordinateur de l'utilisateur. Le nombre de montures disponibles n'est plus limité par la taille des présentoirs de l'opticien mais par la capacité des serveurs. Le client peut donc choisir parmi des milliers de monture à n'importe quelle heure du jour ou de la nuit.

Toutefois, une fois la monture virtuelle choisie, il reste nécessaire de mesurer les paramètres ophtalmiques de l'utilisateur ce qui implique un retour du client chez l'opticien où il va devoir porter une paire de lunettes réelle équivalente à la paire de lunettes virtuelle.

Des dispositifs permettant d'éviter ce passage chez l'opticien ont déjà été proposés. On a, par exemple, envisagé de calculer directement les paramètres ophtalmiques sur la base de la scène 3D créé lors de l'essayage virtuelle. Cependant, la position des lunettes virtuelles dans la scène 3D est grossière et ne permet pas d'effectuer des calculs avec précision.

Pour une même monture, chaque porteur va placer ses lunettes de manière différente en fonction de la position de ses oreilles, de la forme de son nez et des hauteurs de ses pommettes. Il est donc impossible d'utiliser cette scène en l'état pour le calcul des paramètres ophtalmiques.

La présente invention se propose de résoudre ce problème en offrant un procédé qui permet le calcul précis des paramètres ophtalmiques d'un porteur de monture simultanément à l'essayage virtuel de ces montures. Le procédé selon l'invention permet également d'améliorer la précision de la position de la monture virtuelle lors de l'essayage.

### Résumé de l'invention

La présente invention concerne un procédé d'essayage de lunettes virtuelles comprenant les étapes consistant à :
- disposer sur le visage d'un utilisateur une paire de lunettes réelle,
- mesurer la position de ladite paire de lunettes réelle relativement au visage dudit utilisateur au moyen d'un dispositif de mesure,
- cartographier le visage de l'utilisateur par un moyen de cartographie 3D,
- construire un modèle 3D du visage de l'utilisateur et de ladite paire de lunettes virtuelle prenant en compte la position mesurée à l'étape [200], et
- mesurer les paramètres ophtalmologiques dudit utilisateur à partir du modèle 3D du visage de l'utilisateur construit à l'étape [400]. Les procédés de l'art antérieur consistent à créer un modèle 3D dans lequel les lunettes virtuelles sont disposées à la surface du visage de l'utilisateur de manière arbitraire. Dans l'art antérieur, la partie inférieure du pont de la monture est systématiquement posé sur la partie la plus haute de l'arête nasale avec la face arrière dudit pont au contact avec le front de l'utilisateur. Cette position ne correspond pas forcément à la position utilisée habituellement par l'utilisateur et ne permet pas de calculer les paramètres ophtalmiques de manière précise.

Au contraire, dans le procédé selon l'invention, le modèle 3D du visage de l'utilisateur et de ladite paire de lunettes virtuelle est créé en tenant compte de la façon dont l'utilisateur porte en condition normale une paire de lunettes réelle. En conséquence, quelle que soit la paire de lunettes virtuelle, cette dernière sera placée dans le modèle 3D exactement comme l'utilisateur porterait la paire de lunettes réelle équivalente. Ainsi, il est possible de calculer avec précision les paramètres ophtalmiques directement depuis le modèle 3D.

Par l'expression « prenant en compte la position mesurée à l'étape [200] » on entend indiquer que dans le modèle 3D construit à l'étape [400] la position de la paire de lunettes virtuelle, par rapport à la représentation du visage de l'utilisateur, reproduit en tout ou partie celle mesurée à l'étape [200]. Préférentiellement, dans le modèle 3D construit à l'étape [400] la position de la paire de lunettes virtuelle, par rapport à la représentation du visage de l'utilisateur, reproduit en tout point celle mesurée à l'étape [200].

Selon un mode de réalisation préféré, la cartographie du visage de l'utilisateur de l'étape [300] est réalisée après avoir retiré ladite paire de lunettes réelle du visage de l'utilisateur.

Selon un mode de réalisation préféré, le procédé selon l'invention comprend en outre les étapes consistant à :
- saisir l'image du visage de l'utilisateur par un moyen de saisie d'images,
- identifier sur l'image prise à la position [310] la position des pupilles dudit utilisateur,
- intégrer la position des pupilles, déterminées à l'étape [320] au modèle 3D établi à l'étape [400].

Dans le cadre de la présente invention, la position des pupilles peut être remplacée par la position des reflets cornéens. Ainsi, les termes « position des pupilles » et « position des reflets cornéens » peuvent être utilisée de façon interchangeable.

Selon un mode de réalisation préféré, le procédé selon l'invention comprend en outre l'étape [210] consistant à sauvegarder ladite position de ladite paire de lunettes réelle relativement au visage dudit utilisateur, associée à un identifiant spécifique dudit utilisateur.

Selon un mode de réalisation préféré, le procédé selon l'invention comprend en l'étape consistant à [600] afficher, sur un moyen de visualisation, une paire de lunette virtuelle sur l'image du visage dudit utilisateur saisie à l'étape [310], caractérisé en ce que ladite paire de lunettes virtuelle est positionnée de sorte à reproduire la position mesurée à l'étape [200].

Dans le cadre de ce dernier mode de réalisation, l'étape [200] peut être réalisée par l'utilisateur ou par une tierce personne qui va comparer l'emplacement de la paire de lunettes virtuelle affichée à l'étape [600] à la position de la paire de lunettes réelle que l'utilisateur porte, et fournir des informations pour faire coïncider sur ledit moyen de visualisation ladite paire de lunettes réelle et ladite paire de lunettes virtuelle.

Selon un mode de réalisation préféré, les paramètres ophtalmologiques mesurés à l'étape [340] sont choisis dans le groupe consistant en les hauteurs pupillaires, l'angle pantoscopique, l'horizontalité de la monture, l'horizontalité des verres par rapport à ladite monture, les écarts pupillaires en vision de loin et/ou en vision de près, le galbe de ladite monture et la position des repères de centrage sur chacun des verres.

Selon un mode de réalisation préféré, la mesure de la position de ladite paire de lunettes réel relativement au visage dudit utilisateur comprend la détermination du point de contact entre ladite paire de lunettes réel et l'arête du nez dudit utilisateur.

Selon un mode de réalisation préféré, les paramètres ophtalmologiques mesuré à l'étape [340] sont choisis dans le groupe consistant en les hauteurs pupillaires, l'angle pantoscopique, l'horizontalité de la monture, l'horizontalité des verres par rapport à ladite monture, les écarts pupillaires en vision de loin et/ou en vision de près, la distance verre-œil, le galbe de ladite monture, le coefficient tête-œil et la position des repères de centrage sur chacun des verres.

Selon un mode de réalisation préféré, la mesure de la position de ladite paire de lunettes réelle relativement au visage dudit utilisateur comprend la détermination du ou des points de contact entre ladite paire de lunettes réelle et l'arête du nez dudit utilisateur.

Selon un mode de réalisation encore plus préféré, la mesure de la position de ladite paire de lunettes réelle sur le visage dudit utilisateur comprend en outre la détermination du ou des points de contact entre ladite paire de lunettes réelle et chacune des oreilles dudit utilisateur.

Selon un mode de réalisation préféré, ledit dispositif de mesure utilisé à l'étape [200] comprend un moyen apte à mesurer la distance.

Selon un mode de réalisation préféré, ledit moyen apte à mesurer la distance est un capteur ultrasons, une caméra à temps de vol, une caméra truedepth, un LIDAR.

Selon un mode de réalisation préféré, ledit moyen apte à mesurer la distance est associé à un moyen de calcul apte à construire une carte de profondeur du visage de l'utilisateur et de la paire de lunettes réelle.

Selon un mode de réalisation préféré, ledit moyen de saisie d'images utilisé à l'étape [310] est un capteur vidéo.

Selon un mode de réalisation préféré, ledit moyen de visualisation utilisé à l'étape [600] est un écran vidéo.

Selon un mode de réalisation préféré, ledit moyen de saisie d'images et ledit moyen de visualisation sont compris dans une tablette ou un smartphone.

Selon un mode de réalisation préféré, ledit dispositif de mesure, ledit moyen de saisie d'images et ledit moyen de visualisation sont compris dans une tablette.

La présente invention concerne également, un dispositif comprenant un capteur de saisie d'images, un moyen de visualisation, un dispositif de mesure et un moyen de calcul mettant en œuvre un programme informatique mettant en œuvre un procédé selon l'invention.

Les étapes du procédé selon l'invention peuvent être mises en œuvre dans un ordre différent de celui présenté ci-dessus. Par exemple, l'étape [300] peut être réalisé préalablement à l'étape [100] ou à l'étape [200].

### Description des modes de réalisation

Dans le cadre de la présente invention, l'étape [100] consiste à disposer sur le visage d'un utilisateur une paire de lunettes réelle.

Par « paire de lunettes réelle », on entend désigner toute armature composée d'une face et de deux branches. Ladite face comprend en outre un pont destiné à reposer sur le nez de l'utilisateur. La présence de verres au niveau de ladite face n'est pas obligatoire. Le terme « réelle » est utilisé en opposition au terme « virtuel ». Ainsi, ladite paire de lunettes réelle est constituée de matériaux physiques et peut-être touchée.

Ladite paire de lunettes réelle n'est pas obligatoirement une paire de lunettes destinées à recevoir des verres correcteurs. Il peut s'agir, par exemple, d'une structure construite uniquement pour être utilisée dans le cadre du procédé selon l'invention.

Selon un mode de réalisation, ladite paire de lunettes réelle est choisie dans le groupe constitué de l'ensemble des montures disponibles dans le commerce.

Selon un autre mode de réalisation préféré de l'invention, ladite paire de lunettes réelle est une monture de lunette à la surface de laquelle sont disposés, de manière temporaire ou définitives, des repères de tailles et de positions prédéfinies. La présence de ces repères permet de faciliter la détection de la monture et sa position. Ces repères peuvent être, par exemple, des formes géométriques d'une couleur différente de celles du reste de la monture.

Dans le cadre de la présente invention, le terme « position de ladite paire de lunettes réelle relativement au visage dudit utilisateur » entend au moins désigner l'emplacement, sur le nez de l'utilisateur, du ou des points de contact entre le pont de ladite paire de lunettes réelle et le nez de l'utilisateur. Préférentiellement, ladite position comprend en outre l'emplacement, sur les oreilles de l'utilisateur, du ou des points de contact entre les branches de ladite paire de lunettes réelle et le nez de l'utilisateur. Encore plus préférentiellement, ladite position comprend en outre l'emplacement, sur les branches de la paire de lunettes réelle, du ou des points de contact entre les branches de ladite paire de lunettes réelle et les oreilles de l'utilisateur.

Ladite position est définie dans l'espace par rapport à un repère. La nature dudit repère est sans importance dans le cadre de la présente invention. Par exemple, un repère (*O, i̅*, *j̅, k̅*) peut être défini en utilisant quatre points fixes du visage de l'utilisateur. Avantageusement, ces points fixes sont choisis dans le groupe constitué par le coin intérieur de l'œil gauche, le coin intérieur de l'œil droit, le coin extérieur de l'œil gauche, le coin extérieur de l'œil droit, la pointe du nez et la pointe du menton de l'utilisateur.

Ledit « dispositif de mesure » peut être choisi parmi les nombreux dispositifs accessibles à l'homme du métier. Par exemple, ledit dispositif de mesure peut être un ensemble de moyens manuels de prises de mesure tels que des règles, compas et/ou pieds à coulisse.

Dans le cas où le procédé selon l'invention comprend l'étape [600] consistant à afficher, sur un moyen de visualisation, une paire de lunettes virtuelle sur l'image du visage dudit utilisateur saisie à l'étape [310], ledit dispositif de mesure peut comprendre une interface sur laquelle l'utilisateur ou une tierce personne va pouvoir entrer des informations destinées à faire coïncider sur ledit moyen de visualisation ladite paire de lunettes réelle et ladite paire de lunettes virtuelle.

Ladite interface peut notamment être un clavier, un joystick, une souris ou un écran tactile. Selon un mode de réalisation préféré de l'invention, ladite interface est un écran tactile qui sert de moyen de visualisation à l'étape [600].

Dans le cadre de ce mode de réalisation, un premier modèle 3D du visage de l'utilisateur et de ladite paire de lunettes virtuelle, positionnée arbitrairement, est réalisée à partir de la cartographie 3D obtenue à l'étape [300]. Par arbitrairement, on entend indiquer que ladite paire de lunettes virtuelles peut être disposée indifféremment à n'importe quel endroit du premier modèle 3D. Toutefois, avantageusement, ladite paire de lunettes virtuelles est disposée de sorte qu'au moins le pont de ladite paire de lunettes virtuelles soit au contact de l'arête du nez du visage de l'utilisateur.

Ladite paire de lunettes virtuelle est ensuite affichée, sur ledit moyen de visualisation, sur l'image du visage dudit utilisateur saisie à l'étape [310].

Ainsi, l'utilisateur ou une tierce personne va pouvoir comparer l'emplacement de la paire de lunettes virtuelle affichée à celle de la paire de lunettes réelle que l'utilisateur porte, et entrer via ladite interface des informations de correction (e.g. vers le haut, vers le bas, rotation d'avant vers l'arrière, rotation gauche-droite). Lesdites informations de corrections sont utilisées pour corriger la position de la paire de lunettes virtuelle dans ledit modèle 3D. Lorsque l'affichage de la paire de lunettes réelle et celui de la paire de lunettes virtuelle coïncide parfaitement, il peut être admis que la position de la paire de lunettes virtuelle dans le modèle 3D correspond à la position de ladite paire de lunettes réelle relativement au visage dudit utilisateur telle que recherchée à l'étape [200].

Avantageusement, ledit dispositif de mesure utilisé à l'étape [200] est un moyen apte à créer une cartographie 3D du visage de l'utilisateur et de la paire de lunettes réelle portée. La cartographie 3D peut être obtenue par un jeu de plusieurs composants d'équipement travaillant conjointement pour balayer l'environnement continuellement et traiter le signal obtenu pour établir ladite cartographie 3D.

Dans le cadre de l'étape [200], le terme « visage de l'utilisateur » fait référence à au moins une partie de la face avant du crane de l'utilisateur comprenant au moins les yeux, l'arête du nez et le point de jonction entre la partie supérieure de l'oreille et le reste du crâne. Avantageusement, le visage de l'utilisateur cartographié à l'étape [200] comprend au moins la zone du visage délimitée par le haut du front, le menton et l'arrière des oreilles de l'utilisateur.

Ledit moyen apte à créer une cartographie 3D du visage de l'utilisateur peut être n'importe quel type de scanner 3D adapté à la numérisation d'une partie du corps d'un individu. Par exemple, le scanner 3D peut être un scanner 3D à main. Dans certains modes de réalisation, le scanner 3D utilise au moins deux images 2D pour créer le balayage 3D. Par exemple, le scanner 3D peut comprendre plusieurs caméras qui capturent plusieurs images 2D du visage du patient. Le scanner 3D superpose et assemble ensuite les images 2D. Chaque point sur les images 2D est triangulé mathématiquement pour reconstruire l'échelle et l'emplacement dans l'espace afin de créer le scan 3D. Plus le nombre d'images 2D superposées est élevé, plus la résolution et la fiabilité du balayage 3D résultant sont grandes.

Dans d'autres modes de réalisation, le scanner 3D peut comprendre une seule caméra ainsi qu'un équipement supplémentaire qui lui permet de capturer correctement plusieurs images du visage de l'utilisateur qui peuvent être utilisées pour créer une image 3D du visage de l'utilisateur. Par exemple, le scanner 3D peut comprendre une caméra ainsi que des miroirs disposés de telle sorte que la caméra puisse capturer plusieurs images du visage de l'utilisateur. Dans un autre exemple, le scanner 3D peut comprendre une seule caméra ainsi qu'un mécanisme permettant de déplacer la caméra vers un autre emplacement de sorte que la caméra puisse capturer plusieurs images du visage de l'utilisateur à partir de différents emplacements. Dans un autre exemple encore, le scanner 3D peut comprendre une seule caméra ainsi qu'une interface interactive qui indique à l'utilisateur, au moyen d'indicateurs ou d'autres mécanismes, comment déplacer la caméra pour capturer plusieurs images.

Dans un autre mode de réalisation, le scanner 3D peut comprendre une seule caméra ainsi qu'un projecteur de lumière structurée. Le projecteur de lumière structurée peut projeter une grille ou un motif sur le visage de l'utilisateur qui peut être recueilli par la caméra et décodé pour fournir des distances dimensionnelles absolues de l'objet capturé afin de créer un balayage 3D du visage de l'utilisateur.

Dans ce dernier mode de réalisation, ledit moyen apte à créer une cartographie 3D utilisé à l'étape [200] peut comprendre un premier moyen de projection apte à projeter un motif de rayonnement optique sur le visage de l'utilisateur. Selon un mode de réalisation préféré, le rayonnement optique utilisé à cette fin est typiquement dans la plage infrarouge (IR).

Afin de capturer une image du patient avec le motif de rayonnement optique ledit moyen apte à créer une cartographie 3D utilisé à l'étape [200] comprend en outre une première caméra. De préférence, l'axe optique du premier moyen de projection est parallèle à l'axe optique de ladite première caméra. De plus, ladite première caméra comprend un capteur apte à enregistrer le rayonnement optique émis par ledit premier moyen de projection.

Afin de traiter l'image enregistrée par ladite première caméra, ledit moyen apte à créer une cartographie 3D utilisé à l'étape [200] comprend en outre un dispositif de traitement apte à traiter l'image du motif afin de générer une cartographie 3D.

Selon un mode de réalisation préféré de l'invention, ledit dispositif de mesure comprend un moyen apte à mesurer la distance choisi dans le groupe constitué par les capteurs ultrasons, les caméras à temps de vol, les caméras truedepth et les LIDAR.

Selon un mode de réalisation encore plus préféré de l'invention, ledit moyen apte à mesurer la distance est associé à un moyen de calcul apte à construire une carte de profondeur du visage de l'utilisateur et de la paire de lunettes réel.

De plus, le moyen apte à créer une cartographie 3D est associée avantageusement à un dispositif de traitement apte à extraire de ladite cartographie 3D l'emplacement, sur le nez de l'utilisateur, du ou des points de contact entre le pont de ladite paire de lunettes réelle et le nez de l'utilisateur et avantageusement l'emplacement, sur les oreilles de l'utilisateur, du ou des points de contact entre les branches de ladite paire de lunettes réelle et le nez de l'utilisateur et encore plus avantageusement l'emplacement, sur les branches de la paire de lunettes réelle, du ou des points de contact entre les branches de ladite paire de lunettes réel et le nez de l'utilisateur.

Selon un mode de réalisation préféré, le dispositif de traitement est apte à identifier des parties du visage du patient telles que le coin interne des yeux, le centre des pupilles, le milieu du front, et/ou le milieu du menton et déterminer leur emplacement en 3D.

Ce jeu d'équipement apte à produire une cartographie 3D est fourni, par exemple, dans un smartphone ou une tablette. À cet égard, le procédé selon l'invention est mis en œuvre par un smartphone ou une tablette. Dans ce mode de réalisation, le procédé selon l'invention est plus particulièrement mis en œuvre par un logiciel dédié à un smartphone ou une tablette.

Selon un mode de réalisation préféré, le procédé selon l'invention comprend en outre l'étape [210] consistant à sauvegarder ladite position associée à un identifiant spécifique dudit utilisateur, dans une base de données.

Par « identifiant spécifique », on entend désigner tout moyen unique ou pluriel permettant d'identifier un utilisateur. Parmi ceux-ci on peut notamment citer l'état-civil, le numéro de sécurité social, le numéro de passeport, l'adresse, la date de naissance, le numéro client, les données anthropométriques et leurs combinaisons. Selon un mode de réalisation préféré, ledit identifiant spécifique est une donnée anthropométrique de l'utilisateur et encore plus préférentiellement une donnée anthropométrique obtenue à partir de la cartographie 3D de son visage. Tout autre identifiant spécifique peut être créé pour être utilisé dans le cadre du procédé selon l'invention.

Par « sauvegarde », on entend la conservation desdites informations sur tout support de stockage. Les supports de stockage adaptés sont, par exemple, les supports d'informations électroniques et électromagnétiques physiques (e.g. disque dur) ou virtuel (e.g. cloud). Selon un mode de réalisation préféré de l'invention, ledit support de stockage est accessible à distance via le réseau internet.

Ledit identifiant spécifique est associée à ladite position de telle sorte à ce que la lecture du support de stockage permet de retrouver la position de ladite paire de lunettes réelle relativement au visage d'un utilisateur spécifique.

L'étape [300] consistant à cartographier le visage de l'utilisateur, après avoir retiré ladite paire de lunettes réelle, par un moyen de cartographie 3D, peut être réalisé par un moyen identique ou différent dudit dispositif de mesure utilisé à l'étape [200].

Selon un mode de réalisation préféré, le procédé selon l'invention comprend en outre l'étape [310] consistant à saisir l'image du visage de l'utilisateur, après avoir retiré ladite paire de lunettes réel, par un moyen de saisie d'images.

Par « moyen de saisie d'images » on entend préférentiellement désigner un capteur photographique (e.g. CCD, CMOS). Ledit capteur est associé à des moyens de traitement du signal dudit capteur apte à créer une image numérique. Lorsque le dispositif de mesure utilisé à l'étape [200] comprend un moyen de saisie d'image, ce dernier peut être identique ou différent dudit moyen de saisie d'images utilisé à l'étape [310].

La saisie de l'image du visage de l'utilisateur est faite ponctuellement ou en continue.

Ledit moyen de saisie d'image est fourni, par exemple, dans un smartphone ou une tablette. À cet égard, le procédé selon l'invention est mis en œuvre par un smartphone ou une tablette. Dans ce mode de réalisation, le procédé selon l'invention est plus particulièrement mis en œuvre par un logiciel dédié à un smartphone ou une tablette.

Selon un mode de réalisation préféré, le procédé selon l'invention comprend en outre l'étape [320] consistant à identifier sur l'image du visage de l'utilisateur prise à l'étape [310] la position des pupilles dudit utilisateur. Différents algorithmes peuvent être mis en œuvre pour la réalisation de cette étape. Ces algorithmes peuvent mettre en œuvre des étapes successives consistant à détecter le visage de l'utilisateur, puis à détecter les yeux de l'utilisateur dans la zone identifiée précédemment et finalement à détecter les pupilles de l'utilisateur. Parmi ces algorithmes on peut citer ceux décrits par Ciesla et al. (Ciesla, M., & Koziol, P. (2012). Eye Pupil Location Using Webcam. ArXiv, abs/ 1202.6517.).

Dans l'étape [340], la position des pupilles, déterminées à l'étape [320] est intégrée à au modèle 3D établi à l'étape [400].

Par « intégrée » on entend signifier que ladite position 2D des pupilles mesurées à partir de l'image saisie à l'étape [310] est transformée en une position 3D dans le référentiel de la cartographie 3D établie à l'étape [300] puis intégrée dans le modèle 3D. Cette opération peut s'effectuer simplement en prenant en compte les positions 3D des coins des yeux et du sommet de la cornée.

Selon un mode de réalisation préféré, le procédé selon l'invention comprend en outre l'étape [400] consistant à construire un modèle 3D du visage de l'utilisateur et de ladite paire de lunettes virtuelle.

La construction d'un modèle 3D du visage de l'utilisateur et de ladite paire de lunettes virtuelle effectuée à l'étape [400] peut être réalisée par tout système de modélisation 3D connu de l'homme du métier. Cette construction va conduire à l'obtention d'une scène 3D (voir par exemple https://fr.wikipedia.org/wiki/Scéne_3D) comprenant au moins la face du visage de l'utilisateur et la paire de lunettes virtuelle choisie.

Les logiciels permetta nt la construction d'un modèle 3D sont bien connus de l'homme du métier, parmi ceux-ci on peut notamment citer Ogre3D, Autodesk 3ds Max, Autodesk Maya, MotionBuilder, Autodesk Softimage, Bryce 3D, Carrara, Cinema 4D, Dynamation, Houdini, LightWave 3D, MASSIVE, Messiah, Moviestorm, Softimage 3D, Strata 3D, Swift 3D.

Dans le cadre de la présente invention, ladite construction d'un modèle 3D peut ne pas comprendre la mise en œuvre d'un moteur de rendu. En effet, le but premier de cette modélisation est le calcul des positions relatives des éléments de ladite paire de lunettes virtuelles vis-à-vis du visage de l'utilisateur. Il n'est donc pas nécessaire de calculer le rendu de cette scène et de l'afficher (sauf dans le cas où le procédé selon l'invention comprend en outre l'étape [600]).

Dans le cadre de la présente invention, le terme « ladite paire de lunettes virtuelle » fait référence à une paire de lunettes qui est affichée dans l'environnement virtuel mais ne fait pas partie du monde physique.

De nombreuses paires de lunettes virtuelles sont disponibles commercialement. Il existe de nombreuses bases de données contenant plusieurs milliers de lunettes virtuelle dans lequel l'utilisateur peut choisir préalablement à l'étape [400].

Dans la pratique, une paire de lunettes virtuelle est définie par une collection de points dans l'espace 3D, reliés par diverses entités géométriques telles que des triangles, des lignes, des surfaces courbes, etc. Cette collection de points peut être complétée par des informations relatives à la couleur, à la texture et à la réflectance des différentes surfaces de ladite paire de lunettes virtuelle. Étant une collection de données (points et autres informations), les paires de lunettes virtuelle peuvent être créés manuellement, de manière algorithmique ou par balayage. Cet ensemble d'information peut être réuni dans un ou plusieurs fichiers informatiques. Avantageusement, lesdits fichiers sont conservés sur un ou plusieurs supports de stockage. Préférentiellement, ledit support de stockage est accessible à distance via le réseau internet.

Lorsque le procédé selon l'invention ne comprend pas d'étape [600] ladite paire de lunettes virtuelle peut ne pas comprendre de textures, puisque seule la forme géométrique 3D de ladite paire de lunettes virtuelles est nécessaire au calcul des paramètres ophtalmiques.

L'étape [500] consiste à mesurer les paramètres ophtalmologiques de l'utilisateur à partir du modèle 3D du visage de l'utilisateur construit à l'étape [400]. Parmi les paramètres ophtalmologiques mesuré à l'étape [500] on peut citer les hauteurs pupillaires, l'angle pantoscopique, l'horizontalité de la monture, l'horizontalité des verres par rapport à ladite monture, les écarts pupillaires en vision de loin et/ou en vision de près, la distance verre-œil, le galbe de ladite monture, le coefficient tête-œil, la distance verre-oeil et la position des repères de centrage sur chacun des verres.

Par exemple, à partir du modèle 3D calculé à l'étape [400] il est aisé de calculer les hauteurs pupillaires à partir de la position de la pupille de l'utilisateur par rapport à celle du cerclage de la monture encadrant le verre.

De la même manière, la distance verre-œil peut être calculé à partir de la position, dans le modèle 3D, du sommet de la cornée et des verres (ou de leur position déduite à partir de la position du cerclage).

L'angle pantoscopique peut être calculée en mesurant l'angle de la face de la paire de lunettes virtuelle par rapport au plan vertical.

L'ensemble de ces calculs peut être fait avec l'aide d'un opérateur qui peut désigner, via une interface informatique, les différents points (sommet de la cornée, position des verres, etc...) nécessaires au calcul des paramètres ophtalmiques. Le calcul peut être avantageusement automatisé à partir des points identifiés par l'opérateur.

Alternativement, l'ensemble de l'étape [500] est réalisé automatiquement par un moyen de traitement d'information programmé de sorte à identifier à l'intérieur dudit modèle 3D les points nécessaires au calcul des paramètres ophtalmiques et au calcul de ces derniers.

L'étape [600] consistant à afficher, sur un moyen de visualisation, une paire de lunette virtuelle sur l'image du visage dudit utilisateur saisie à l'étape [310], caractérisé en ce que ladite paire de lunettes virtuelle est positionné de sorte à reproduire la position mesurée à l'étape [200].

Dans le cadre de la présente invention, le terme « moyen de visualisation » entend référer à tout moyen permettant l'affichage d'images et plus particulièrement d'images numériques.

La création d'une image numérique comprenant une paire de lunette virtuelle sur l'image du visage dudit utilisateur à partir dudit modèle 3D créé à l'étape [400] est une étape classique de l'infographie 3D. Préférentiellement, l'étape [500] met en œuvre en moteur de rendu 3D. Un moteur de rendu 3D peut être un logiciel classique ou des algorithmes intégrés dans des cartes graphiques spéciales qui calculent une ou plusieurs images 3D en y restituant non seulement la projection 3D, les textures et les effets d'éclairage.

Le moteur de rendu 3D analyse les éléments d'une image numérisée (couleurs, intensité et type de la lumière, ombres et leurs combinaisons, etc.), image censée être vue par une « caméra » virtuelle dont les coordonnées x y z déterminent l'angle de vue et la position des objets.

Parmi, les moteurs de rendu 3D utilisables dans le cadre de la présente invention, on peut notamment citer Arnold, Aqsis, Arion Render, Artlantis, Atomontage1, Blender, Brazil r/s, BusyRay, Cycles, Enscape, FinalRender, Fryrender, Guerilla Render2, Indigo, Iray, Kerkythea, KeyShot, Kray, Lightscape, LightWorks, Lumiscaphe, LuxRender, Maxwell Render, Mental Ray, Mitsuba3, Nova, Octane4, POV-Ray, RenderMan, Redsdk, Redway3d, Sunflow, Turtle, V-Ray, VIRTUALIGHT et YafaRay.

De nombreux procédés permettant de mettre en œuvre l'étape [600] consistant à afficher, sur un moyen de visualisation, une paire de lunette virtuelle sur l'image du visage dudit utilisateur saisie à l'étape [310] sont décrits dans l'art antérieur. Parmi ceux-ci on peut notamment citer les procédés décrits dans les documents WO013207 et EP2526510 ou les logiciels commerciaux tels que SmartMirror^{®}.

L'ensemble du procédé selon l'invention peut être mis en œuvre par un logiciel implémenté dans un smartphone, une tablette ou un ordinateur.

Ainsi la présente invention concerne également un smartphone, une tablette ou un ordinateur remarquable en ce qu'il met en œuvre un procédé selon l'invention.

## Revendications

1. Procédé d'essayage de lunettes virtuelles comprenant les étapes consistant à :
- [100] disposer sur le visage d'un utilisateur une paire de lunettes réelle,
- [200] mesurer la position de ladite paire de lunettes réelle relativement au visage dudit utilisateur au moyen d'un dispositif de mesure,
- [300] cartographier le visage de l'utilisateur par un moyen de cartographie 3D,
- [400] construire un modèle 3D du visage de l'utilisateur et de ladite paire de lunettes virtuelle prenant en compte la position mesurée à l'étape [200], et
- [500] mesurer les paramètres ophtalmologiques dudit utilisateur à partir du modèle 3D du visage de l'utilisateur construit à l'étape [400].

2. Procédé d'essayage de lunettes virtuelles selon la revendication 1 comprenant en outre les étapes consistant à :
- [310] saisir l'image du visage de l'utilisateur par un moyen de saisie d'images,
- [320] identifier, sur l'image prise à la position [310], la position des pupilles dudit utilisateur,
- [340] intégrer la position des pupilles, déterminées à l'étape [320] au modèle 3D établi à l'étape [400].

3. Procédé d'essayage de lunettes virtuelles selon la revendication 1 comprenant en outre l'étape [210] consistant à sauvegarder ladite position associée à un identifiant spécifique dudit utilisateur.

4. Procédé d'essayage de lunettes virtuelles selon la revendication 2 comprenant en outre l'étape [600] consistant à afficher, sur un moyen de visualisation, une paire de lunette virtuelle sur l'image du visage dudit utilisateur saisie à l'étape [310], **caractérisé en ce que** ladite paire de lunettes virtuelle est positionnée de sorte à reproduire la position mesurée à l'étape [200].

5. Procédé d'essayage de lunettes virtuelles selon l'une des revendications précédentes **caractérisé en ce que** les paramètres ophtalmologiques mesuré à l'étape [500] sont choisis dans le groupe consistant en les hauteurs pupillaires, l'angle pantoscopique, l'horizontalité de la monture, l'horizontalité des verres par rapport à ladite monture, les écarts pupillaires en vision de loin et/ou en vision de près, le galbe de ladite monture, la distance verre-oeil et la position des repères de centrage sur chacun des verres.

6. Procédé d'essayage de lunettes virtuelles selon l'une des revendications précédentes **caractérisé en ce que** la mesure de la position de ladite paire de lunettes réel relativement au visage dudit utilisateur comprend la détermination du point de contact entre ladite paire de lunettes réel et l'arête du nez dudit utilisateur.

7. Procédé d'essayage de lunettes virtuelles selon l'une des revendications précédentes **caractérisé en ce que** la mesure de la position de ladite paire de lunettes réel sur le visage dudit utilisateur comprend la détermination du point de contact entre ladite paire de lunettes réel et chacune des oreilles dudit utilisateur.

8. Procédé d'essayage de lunettes virtuelles selon l'une des revendications précédentes **caractérisé en ce que** ledit dispositif de mesure comprend un moyen apte à mesurer la distance.

9. Procédé d'essayage de lunettes virtuelles selon la revendication 8 **caractérisé en ce que** ledit moyen apte à mesurer la distance est un capteur ultrasons, une caméra à temps de vol, une caméra truedepth, un LIDAR.

10. Procédé d'essayage de lunettes virtuelles selon la revendication 8 **caractérisé en ce que** ledit moyen apte à mesurer la distance est associé à un moyen de calcul apte à construire une carte de profondeur du visage de l'utilisateur et de la paire de lunettes réel.

11. Procédé d'essayage de lunettes virtuelles selon l'une des revendications précédentes **caractérisé en ce que** ledit moyen de saisie d'images est un capteur vidéo.

12. Procédé d'essayage de lunettes virtuelles selon la revendication 11 **caractérisé en ce que** ledit moyen de visualisation est un écran vidéo.

13. Procédé d'essayage de lunettes virtuelles selon l'une des revendications précédentes **caractérisé en ce que** ledit moyen de saisie d'images et ledit moyen de visualisation sont compris dans une tablette.

14. Procédé d'essayage de lunettes virtuelles selon l'une des revendications précédentes **caractérisé en ce que** ledit dispositif de mesure, ledit moyen de saisie d'images et ledit moyen de visualisation sont compris dans une tablette.

## Patentansprüche

1. Verfahren zum Anprobieren einer virtuellen Brille, umfassend folgende Schritte:
- [100] Anlegen einer echten Brille auf das Gesicht eines Benutzers,
- [200] Messen der Position der Brille relativ zum Gesicht des Benutzers mittels einer Messvorrichtung,
- [300] Kartieren des Gesichts des Benutzers mit einem 3D-Kartierungsmittel,
- [400] Erstellen eines 3D-Modells des Gesichts des Benutzers und der virtuellen Brille unter Berücksichtigung der in Schritt [200] gemessenen Position, und
- [500] Messen der augenärztlichen Parameter des Benutzers aus dem in Schritt [400] gebauten 3D-Modells des Gesichts des Benutzers.

2. Verfahren zum Anprobieren einer virtuellen Brille nach Anspruch 1, ferner umfassend die Schritte zum:
- [310] Erfassen des Bildes des Gesichts des Benutzers mit einem Bilderfassungsmittel,
- [320] Identifizieren, auf dem in Position [310] aufgenommenen Bild, der Position der Pupillen des Benutzers,
- [340] Integrieren der in Schritt [320] ermittelten Position der Pupillen in das in Schritt [400] erstellte 3D-Modell.

3. Verfahren zum Anprobieren einer virtuellen Brille nach Anspruch 1, ferner umfassend den Schritt [210], der darin besteht, die mit einer spezifischen Kennung des Benutzers verbundene Position zu speichern.

4. Verfahren zum Anprobieren einer virtuellen Brille nach Anspruch 2, ferner umfassend Schritt [600], das darin besteht, auf einem Visualisierungsmittel eine virtuelle Brille auf dem in Schritt [310] erfassten Gesichtsbild des Benutzers anzuzeigen, **dadurch gekennzeichnet, dass** die virtuelle Brille so positioniert ist, dass es die in Schritt [200] gemessene Position reproduziert.

5. Verfahren zum Anprobieren einer virtuellen Brille nach einem der vorhergehenden Ansprüche, **dadurch gekennzeichnet, dass** die in Schritt [500] gemessenen augenärztlichen Parameter aus der Gruppe ausgewählt sind, die aus den Pupillenhöhen, dem Pantoskopwinkel, der Horizontalität der Fassung, der Horizontalität der Gläser in Bezug auf die Fassung, den Pupillenabweichungen bei Fern- und/oder Nahsicht, der Kontur der Fassung, dem Glas-Augen-Abstand und der Position der Zentriermarkierungen auf jedem Glas, besteht.

6. Verfahren zum Anprobieren einer virtuellen Brille nach einem der vorhergehenden Ansprüche, **dadurch gekennzeichnet, dass** das Messen der Position der Brille in Bezug auf das Gesicht des Benutzers die Bestimmung des Kontaktpunkts zwischen der Brille und dem Nasenrand des Benutzers umfasst.

7. Verfahren zum Anprobieren einer virtuellen Brille nach einem der vorhergehenden Ansprüche, **dadurch gekennzeichnet, dass** das Messen der Position der Brille am Gesicht des Benutzers die Bestimmung des Kontaktpunkts zwischen der Brille und jedem der Ohren des Benutzers umfasst.

8. Verfahren zum Anprobieren einer virtuellen Brille nach einem der vorhergehenden Ansprüche, **dadurch gekennzeichnet, dass** die Messvorrichtung ein zur Entfernungsmessung geeignetes Mittel umfasst.

9. Verfahren zum Anprobieren einer virtuellen Brille nach Anspruch 8, **dadurch gekennzeichnet, dass** das zur Entfernungsmessung geeignete Mittel ein Ultraschallsensor, eine Flugzeitkamera, eine Truedepth-Kamera, ein LIDAR ist

10. Verfahren zum Anprobieren einer virtuellen Brille nach Anspruch 8, **dadurch gekennzeichnet, dass** das zur Entfernungsmessung geeignete Mittel mit einem zur Erstellung einer Tiefenkarte des Gesichts des Benutzers und der Brille geeigneten Berechnungsmittel verbunden ist.

11. Verfahren zum Anprobieren einer virtuellen Brille nach einem der vorhergehenden Ansprüche, **dadurch gekennzeichnet, dass** das Bilderfassungsmittel ein Videosensor ist.

12. Verfahren zum Anprobieren einer virtuellen Brille nach Anspruch 11, **dadurch gekennzeichnet, dass** das Visualisierungsmittel ein Videobildschirm ist.

13. Verfahren zum Anprobieren einer virtuellen Brille nach einem der vorhergehenden Ansprüche, **dadurch gekennzeichnet, dass** das Bilderfassungsmittel und das Visualisierungsmittel in einem Tablet enthalten sind.

14. Verfahren zum Anprobieren einer virtuellen Brille nach einem der vorhergehenden Ansprüche, **dadurch gekennzeichnet, dass** die Messvorrichtung, das Bilderfassungsmittel und das Visualisierungsmittel in einem Tablet enthalten sind.

## Claims

1. A method for fitting virtual glasses comprising the steps of:
- [100] placing on the face of a user a real pair of glasses,
- [200] measuring the position of said real pair of glasses relative to the face of said user by means of a measuring device,
- [300] mapping the face of the user by a 3D mapping means,
- [400] constructing a 3D model of the face of the user and of said virtual pair of glasses taking into account the position measured in step [200], and
- [500] measuring the ophthalmological parameters of said user based on the 3D model of the face of the user constructed in step [400].

2. The method for fitting virtual glasses according to claim 1, further comprising the steps of:
- [310] capturing the image of the face of the user by an image capture means,
- [320] identifying, on the image captured in step [310], the position of the pupils of said user,
- [340] integrating the position of the pupils, determined in step [320] to the 3D model established in step [400].

3. The method for fitting virtual glasses according to claim 1, further comprising step [210] of saving said position associated with a specific identifier of said user.

4. The method for fitting virtual glasses according to claim 2, further comprising step [600] of displaying, on a display means, a virtual pair of glasses on the image of the face of said user captured in step [310], **characterised in that** said virtual pair of glasses is positioned so as to reproduce the position measured in step [200].

5. The method for fitting virtual glasses according to one of the preceding claims, **characterised in that** the ophthalmological parameters measured in step [500] are selected from the group consisting of the pupillary heights, the pantoscopic angle, the horizontality of the frame, the horizontality of the lenses with respect to said frame, the pupillary deviations in far-sight and/or near-sight, the camber of said frame, the lens-eye distance and the position of the centring marks on each of the lenses.

6. The method for fitting virtual glasses according to one of the preceding claims, **characterised in that** the measurement of the position of said real pair of glasses relative to the face of said user comprises determining the point of contact between said real pair of glasses and the edge of the nose of said user.

7. The method for fitting virtual glasses according to one of the preceding claims, **characterised in that** the measurement of the position of said real pair of glasses on the face of said user comprises determining the point of contact between said real pair of glasses and each of the ears of said user.

8. The method for fitting virtual glasses according to one of the preceding claims, **characterised in that** said measuring device comprises a means capable of measuring the distance.

9. The method for fitting virtual glasses according to claim 8, **characterised in that** said means capable of measuring the distance is an ultrasound sensor, a time-of-flight camera, a truedepth camera, a LIDAR.

10. The method for fitting virtual glasses according to claim 8, **characterised in that** said means capable of measuring the distance is associated with a computing means capable of constructing a depth map of the face of the user and of the real pair of glasses.

11. The method for fitting virtual glasses according to one of the preceding claims, **characterised in that** said image capture means is a video sensor.

12. The method for fitting virtual glasses according to claim 11, **characterised in that** said display means is a video screen.

13. The method for fitting virtual glasses according to one of the preceding claims, **characterised in that** said image capture means and said display means are included in a tablet.

14. The method for fitting virtual glasses according to one of the preceding claims, **characterised in that** said measuring device, said image capture means and said display means are included in a tablet.
